Europäisches Patentamt

European Patent Office

Office européen des brevets

(11) Numéro de publication : **0 084 286 B1**

(12) **FASCICULE DE BREVET EUROPÉEN**

(45) Date de publication du fascicule du brevet :
18.09.85

(51) Int. Cl.⁴ : **C 07 D313/04**

(21) Numéro de dépôt : 82402394.9

(22) Date de dépôt : **29.12.82**

(54) **Procédé perfectionné de fabrication de l'epsilon-caprolactone.**

(30) Priorité : 15.01.82 FR 8200564

(43) Date de publication de la demande :
27.07.83 Bulletin 83/30

(45) Mention de la délivrance du brevet :
18.09.85 Bulletin 85/38

(84) Etats contractants désignés :
BE CH DE FR GB IT LI LU NL SE

(56) Documents cités :
EP-A- 0 020 952
EP-A- 0 059 655
CH-A- 475 235
FR-A- 1 160 882
FR-A- 2 101 985
JOURNAL OF APPLIED CHEMISTRY, vol. 46, no. 9
septembre 1976, pages 2035-2041, Moscou, SU V.M.
VISHNYAKOV et al.: "Kinetics and mechanism of the
interaction of cyclohexanone with peracetic acid"

(73) Titulaire : ATOCHEM
12/16, allée des Vosges
F-92400 Courbevoie (FR)

(72) Inventeur : Pralus, Michèle
3, rue des Gosses
F-69450 Saint Cyr au Mont d'Or (FR)
Inventeur : Lecoq, Jean-Claude
10 A, rue Josserand
F-69630 Chaponost (FR)
Inventeur : Schirmann, Jean-Pierre
49, chemin de la Glacière
F-69600 Oullins (FR)

(74) Mandataire : Rochet, Michel et al
ATOCHEM Département Propriété Industrielle Cedex
22
F-92091 Paris la Défense (FR)

**Description**

La présente invention concerne un perfectionnement au procédé de préparation de l'ε-caprolactone par oxydation de la cyclohexanone au moyen d'une solution organique brute d'acide percaboxylique.

L'objet de la présente invention est un procédé de fabrication de l'ε-caprolactone par oxydation de la cyclohexanone au moyen d'une solution brute d'acide percarboxylique en $C_2$-$C_4$, résultant de la réaction du peroxyde d'hydrogène sur l'acide carboxylique correspondant en présence d'un acide borique comme catalyseur avec élimination continue de l'eau par entraînement azéotropique, caractérisé en ce que la réaction entre la cyclohexanone et l'acide percarboxylique est réalisée avec un rapport molaire de la cyclohexanone mise en œuvre à l'acide percarboxylique compris entre 0,50 et 0,90, l'acide carboxylique non transformé, l'excès d'acide percarboxylique et le solvant entraîneur azéotropique sont séparés par distillation du mélange réactionnel après l'oxydation et recyclés à l'étape de synthèse de l'acide percarboxylique et l'ε-caprolactone brute résultant de la distillation précédente purifiée par une simple évaporation sous pression réduite.

En particulier, le présent procédé est caractérisé en ce que l'acide percarboxylique mis en œuvre est l'acide perpropionique et que le solvant entraîneur azéotropique est le dichloro-1,2-éthane.

Dans sa demande de brevet français n° 8 103 374 du 20 février 1981, la demanderesse a montré la possibilité d'utiliser directement pour l'oxydation de la cyclohexanone des solutions brutes d'acides percarboxyliques, tels que l'acide perpropionique, lorsque ces solutions sont obtenues selon la technique décrite dans la demande de brevet français n° 2 464 947.

Cette technique consiste à faire réagir le peroxyde d'hydrogène avec un acide carboxylique miscible à l'eau, en présence d'un acide borique comme catalyseur, tout en éliminant en continu l'eau du milieu réactionnel par entraînement azéotropique à l'aide d'un solvant organique inerte, tel que le dichloro-1,2-éthane. Un perfectionnement à cette technique, consistant à injecter de l'eau dans la colonne de distillation azéotropique est décrit dans la demande de brevet français n° 8 200 407 du 13 janvier 1982.

Lorsque les solutions brutes d'acides percarboxyliques ainsi obtenues sont utilisées, selon l'enseignement de la demande de brevet français n° 8 103 374, pour l'oxydation de la cyclohexanone, on utilise un rapport molaire de la cyclohexanone à l'acide percarboxylique compris entre 1 et 5, et de préférence entre 1 et 1,5. On obtient dans ces conditions des rendements en ε-caprolactone de l'ordre de 92 % par rapport à l'oxygène peroxydique mis en œuvre.

L'étude cinétique de la réaction entre la cyclohexanone et l'acide peracétique, effectuée par V.M. VISHNYAKOV et al. (Journal of Applied Chemistry U.S.S.R., 49, n° 9, 2035-1976) a montré qu'il s'agissait d'une réaction bimoléculaire du premier ordre vis-à-vis de chacun des réactifs. Toutefois l'art antérieur concernant l'oxydation de la cyclohexanone par les peracides a toujours préconisé d'utiliser un excès de cétone par rapport au peracide. Cet excès de cétone joue le rôle d'un diluant pour la réaction et évite la formation fâcheuse de peroxydes, qui constituent un danger sérieux pour l'exploitation. Cette formation de peroxyde est même notée selon CH-A-475 235 lorsque la cyclohexanone est oxydée par l'acide performique en léger défaut molaire par rapport à elle.

Des rapports molaires de la cyclohexanone au peracide allant de 2 à 15 ont ainsi été recommandés quel que soit le mode de préparation de l'acide percarboxylique utilisé :

Solutions aqueuses de peracide (demande de brevet japonais 45-15737/1970 et DE-B-1 258 858).

Peracide formé in situ par co-oxydation de la cyclohexanone et de l'acétaldéhyde (K. TANAKA et al., Kogyo Kagaku Zasshi 73, 943, 1970).

Solutions organiques anhydres de peracide (P.S. STARCHER, Journal of American Chemical Society, 80, 4079, 1958 et brevets français n° 1 160 882 et 2 101 985).

Si l'emploi d'un excès molaire de cyclohexanone par rapport à l'acide percarboxylique présente souvent certains avantages du point de vue facilité et sécurité de l'exploitation, cette technique a par contre l'inconvénient de nécessiter après la réaction d'oxydation, la séparation par distillation de la cyclohexanone résiduaire en vue de son recyclage.

Dans le cas où l'acide percarboxylique utilisé est l'acide perpropionique, comme par exemple dans DE-C-2 920 436 de la République Fédérale Allemande, il est en outre nécessaire d'effectuer la séparation par distillation de la cyclohexanone et de l'acide propionique, chacun de ces réactifs étant recyclé à un stade différent du procédé.

L'existence d'un azéotrope sous pression réduite entre la cyclohexanone et l'acide propionique rend cette séparation d'autant plus difficile qu'il s'agit de composés dont les points d'ébullition sont très proches. Par exemple, sous une pression de 100 mm de mercure (13,3 kPa), les points d'ébullition sont les suivants :

| | |
|---|---|
| Acide propionique | 86 °C |
| Cyclohexanone | 93 °C |
| Azéotrope | 93 °C |

Il n'est pas possible d'autre part d'effectuer à la pression ordinaire cette séparation entre l'acide propionique et la cyclohexanone, car, sous l'effet d'une température élevée et aussi sous l'influence de catalyseurs variés, en particulier acides, la cyclohexanone se condense facilement sur elle-même, comme l'a montré C.D. HURD (Journal of American Chemical Society, 61, 3359-1939). Parmi les produits de condensation les plus bas, il se forme notamment un dimère, constitué d'un mélange de deux isomères, la cyclohexène-1-yl-2-cyclohexanone et la cyclo-hexylidène-2-cyclohexanone. Ces sous-produits à point d'ébullition élevé se retrouveraient ensuite comme impuretés dans l'ε-caprolactone.

Poursuivant ses recherches sur les applications des solutions organiques brutes d'acides percarboxyliques obtenues selon la demande de brevet français n° 2 464 947, la demanderesse vient de découvrir que, contrairement à tout ce qu'enseignait l'art antérieur, il est possible d'effectuer la réaction d'oxydation de la cyclohexanone par ces solutions brutes de peracide en utilisant un excès de peracide par rapport à la cyclohexanone, tout en conservant une sélectivité élevée par rapport à la cétone et à l'oxygène peroxydique engagés et en évitant la formation de peroxydes dangereux. Cela n'est possible que parce que la solution organique de peracide utilisée par la demanderesse est pratiquement anhydre et dépourvue de toute trace de catalyseur acide fort, tel que l'acide sulfurique.

Après l'étape d'oxydation, les séparations des différents constituants du mélange réactionnel se font sans perte importante en oxygène peroxydique résiduel. Comme la cyclohexanone engagée est pratiquement entièrement consommée dans la réaction d'oxydation, la fraction de tête obtenue à la distillation des produits bruts d'oxydation peut être recyclée directement à l'étape de synthèse de la solution de peracide. La fraction de pied, constituée essentiellement d'ε-caprolac-tone, est avantageusement purifiée dans une simple distillation ultérieure.

Les avantages du procédé selon l'invention sont donc de conduire de façon simple et efficace à de très bons rendements en ε-caprolactone avec de très faibles consommations d'énergie.

La réaction d'oxydation de la cyclohexanone par la solution brute de peracide est réalisée de préférence à la pression atmosphérique, mais peut tout aussi bien être conduite à des pressions inférieures ou supérieures. La température de réaction est comprise entre 20 et 120 °C, et de préférence entre 40 et 80 °C.

Le rapport molaire de la cyclohexanone mise en œuvre à l'acide percarboxylique est compris entre 0,50 et 0,99, et de préférence entre 0,75 et 0,90.

La réaction peut être réalisée soit en discontinu, soit en continu. Dans ce dernier cas on alimente un réacteur ou plusieurs réacteurs en cascade simultanément avec la cyclohexanone et avec la solution brute d'acide percarboxylique. Le temps de séjour est compris entre 30 minutes et 4 heures, suivant la température choisie pour la réaction.

A la fin de l'oxydation les produits de la réaction sont séparés par distillation selon les techniques usuelles. On récupère d'une part l'acide percarboxylique résiduel et le mélange constitué par l'acide carboxylique et le solvant entraîneur azéotropique, et d'autre part la caprolactone produite. Les distillations sont avantageusement effectuées sous pression réduite, pour limiter les pertes en oxygène peroxydique et la dégradation thermique de l'ε-caprolactone. On utilise de préférence des évaporateurs couramment employés dans l'industrie, tels que les évaporateurs à couche mince ou les évaporateurs à film tombant.

Les exemples suivants illustrent de façon non limitative divers aspects de l'invention. Dans ces exemples, les teneurs en ε-caprolactone et en cyclohexanone des solutions finales sont déterminées par chromatographie en phase gazeuse, tandis que l'oxygène peroxydique résiduaire est dosé chimiquement.

Exemple 1

On prépare une solution brute d'acide perpropionique selon la demande de brevet français n° 2 464 947 de la demanderesse, par action d'une solution aqueuse à 70 % en poids de peroxyde d'hydrogène sur l'acide propionique, en présence de 1 % en poids d'acide orthobori-que, tout en éliminant continuellement l'eau du milieu réactionnel par entraînement azéotropique avec le dichloro-1,2-éthane.

La solution brute obtenue a la composition pondérale suivante :

| | |
|---|---|
| dichloro-1,2-éthane | 16,0 % |
| acide propionique | 59,9 % |
| acide perpropionique | 22,8 % |
| peroxyde d'hydrogène | 0,3 % |
| acide orthoborique | 1,0 % |

On place 150,7 g de cette solution brute d'acide perpropionique dans un réacteur en verre de 250 cm³, muni d'un système d'agitation, surmonté d'un réfrigérant et équipé d'un système de contrôle de la température. Le réacteur est porté à une température de 50 °C, puis on introduit en 30 minutes 33,6 g de cyclohexanone, soit 0,86 mole par mole d'oxygène peroxydique.

Après 3 heures de réaction on arrête l'agitation et refroidit le réacteur. On soutire une solution ayant la composition pondérale suivante :

| | |
|---|---|
| dichloro-1,2-éthane | 13,6 % |
| acide propionique | 62,0 % |
| cyclohexanone | 0,4 % |
| ε-caprolactone | 20,4 % |
| acide perpropionique | 2,8 % |
| acide borique | 0,8 % |

Le taux de conversion de la cyclohexanone est de 98 %. La sélectivité en ε-caprolactone est de 97,5 % par rapport à la cyclohexanone et de 96 % par rapport à l'oxygène peroxydique engagé.

Exemple 2

On répète l'exemple 1, mais en utilisant pour la préparation de la solution brute d'acide perpropionique un mélange récupéré par distillation des produits de réaction d'une précédente opération d'oxydation de la cyclohexanone.

Ainsi, dans un réacteur en verre d'un litre, muni d'un système d'agitation, et équipé d'une colonne de distillation de 15 plateaux OLDERSHAW avec condenseur à reflux, on place 630 g d'une solution ayant la composition pondérale suivante :

| | |
|---|---|
| dichloro-1,2-éthane | 16,4 % |
| acide propionique | 79,5 % |
| cyclohexanone | 0,2 % |
| acide perpropionique | 2,9 % |
| acide borique | 1,0 % |

Cette solution est portée à l'ébullition à reflux sous une pression de 100 mm de mercure (13,3 kPa) et on introduit en 20 minutes 92 g de peroxyde d'hydrogène sous forme d'une solution aqueuse à 70 % en poids. La phase organique de l'hétéro-azéotrope eau-dichloro-1,2-éthane, condensée et décantée, est refluée dans la colonne de distillation. Simultanément on injecte en tête de colonne, pendant 2 heures, 17,5 g/h d'eau, selon le procédé décrit dans la demande de brevet français n° 8 200 407 du 13 janvier 1982. La température dans le réacteur est de 68 °C. Après 2 h 30 de réaction on arrête l'agitation et le chauffage du réacteur. La phase aqueuse provenant de la condensation et de la décantation de l'hétéroazéotrope, soutirée en continu pendant toute la durée de l'opération, pèse 114 g et contient 0,2 % en poids de peroxyde d'hydrogène, soit environ 0,3 % de l'oxygène peroxydique engagé.

La solution brute d'acide perpropionique obtenue contient 26,3 % en poids de peracide et 0,3 % en poids de peroxyde d'hydrogène résiduel.

On engage 600 g de cette solution dans un réacteur en verre d'un litre, muni d'un système d'agitation, d'un réfrigérant et d'un système de régulation de température. On porte la température du réacteur à 50 °C, puis on introduit en 30 minutes 143 g de cyclohexanone, soit 0,8 mole par mole d'oxygène peroxydique engagé.

Après deux heures de réaction on arrête l'agitation et refroidit le réacteur.

La solution obtenue contient principalement 20,8 % en poids d'ε-caprolactone et 4,6 % d'acide perpropionique résiduel.

La sélecticité en ε-caprolactone par rapport à la cyclohexanone est de 99 %.

Exemple 3

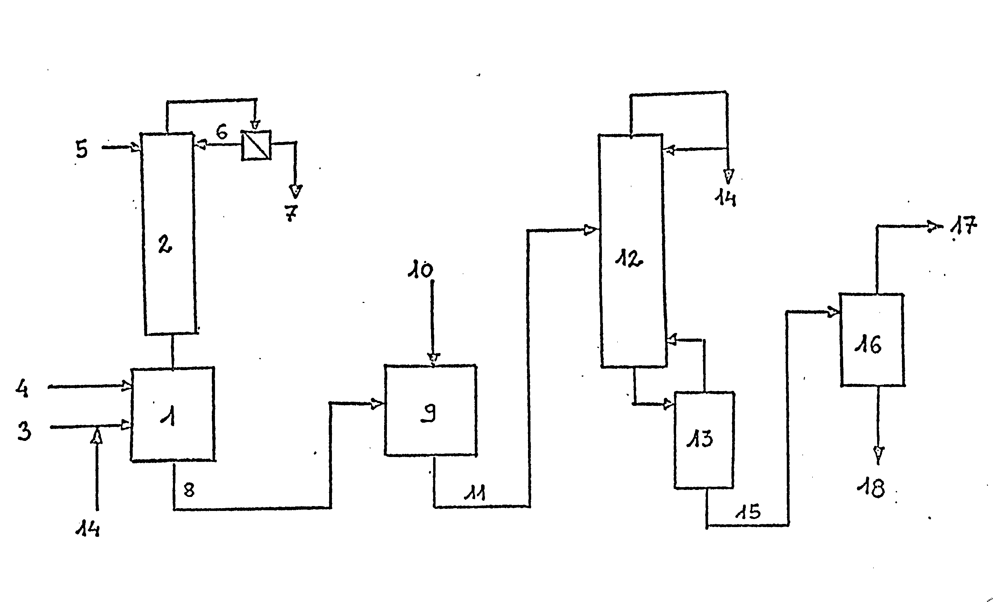

On effectue une opération de préparation d'ε-caprolactone dans l'appareillage schématisé sur la planche unique.

a. Dans un réacteur (1) en verre d'un litre, équipé d'une colonne de distillation (2) de 15 plateaux OLDERSHAW et d'un condenseur à reflux, on introduit 636 g d'une solution (3) ayant la composition pondérale suivante :

| | |
|---|---|
| dichloro-1,2-éthane | 19,8 % |
| acide propionique | 79,2 % |
| acide orthoborique | 1,0 % |

Ce mélange est porté à l'ébullition à reflux sous une pression de 100 mm de mercure (13,3 kPa) et on introduit en 20 minutes 92 g d'une solution aqueuse (4) à 70 % en poids de peroxyde d'hydrogène, contenant également 0,7 % en poids d'acide dipicolinique. Simultanément on injecte en (5), en tête de la colonne de distillation, 21 g/h d'eau pendant 2 heures 30. La température du réacteur est de 65 °C. La phase organique condensée de l'hétéro-azéotrope est recyclée en (6) pour assurer le reflux. La phase aqueuse condensée est décantée et soutirée en continu en (7). On arrête la réaction après 3 heures de marche.

La solution brute de peracide soutirée en (8) contient 23,7 % en poids d'acide perpropionique, ce qui représente un taux de transformation de 92 % par rapport au peroxyde d'hydrogène engagé.

b. La solution brute de peracide est envoyée dans un réacteur (9), où l'on introduit en (10) de la cyclohexanone en quantité telle que le rapport molaire de la cyclohexanone à l'acide perpropionique soit de 0,83. Après 2 h 30 de réaction à 50 °C (ce temps comprenant les 30 minutes de durée de l'introduction de la cyclohexanone), on obtient une solution brute de caprolactone ayant la composition pondérale suivante :

| | |
|---|---|
| ε-caprolactone | 19,8 % |
| dichloro-1,2-éthane | 13,8 % |
| acide propionique | 62,6 % |
| acide perpropionique | 3,4 % |
| cyclohexanone | 0,4 % |

La conversion de la cyclohexanone est donc de 97 %. La sélectivité en ε-caprolactone est de 96 % par rapport à la cyclohexanone et de 92 % par rapport à l'acide perpropionique.

c. Cette solution brute d'ε-caprolactone est introduite en continu, à raison de 365 g/heure, par la canalisation (11) dans une colonne de distillation (12), équipée de 25 plateaux OLDERSHAW, et fonctionnant sous une pression de 10 mm de mercure (1,33 kPa). Cette colonne est équipée d'un évaporateur à film tombant (13), fonctionnant comme bouilleur. La température est de 33 °C en tête de colonne et de 141 °C en pied. On utilise un taux de reflux de 0,15. Après condensation en tête de colonne, on soutire en (14) 290 g/h d'une solution contenant 73,4 % en poids d'acide propionique, 16,3 % en poids de dichloro-1,2-éthane et 4 % en poids d'acide perpropionique. Cette solution peut être recyclée directement à l'étape a) de synthèse du peracide. Le taux de récupération de l'oxygène peroxydique dans cette distillation est de 93 %.

d. On soutire en continu du pied de colonne (15) 75 g/h d'une solution contenant 93,7 % d'ε-caprolactone et moins de 100 ppm de cyclohexanone et moins de 700 ppm d'acide propionique.

Cette solution est purifiée par une simple évaporation sous pression réduite de 10 mm de mercure (1,33 kPa) dans un évaporateur à film tombant (16), identique à l'évaporateur (13). La fraction de pied est éliminée en (18) et l'on recueille en (17) une ε-caprolactone, dont le titre de 99,3 % en poids répond aux spécifications commerciales en usage.

## Revendications

1. Procédé de fabrication de l'ε-caprolactone par oxydation de la cyclohexanone au moyen d'une solution brute d'acide percarboxylique en $C_2$-$C_4$, résultant de la réaction du peroxyde d'hydrogène sur l'acide carboxylique correspondant en présence d'un acide borique comme catalyseur avec élimination continue de l'eau par entraînement azéotropique, caractérisé en ce que la réaction entre la cyclohexanone et l'acide percarboxylique est réalisée avec un rapport molaire de la cyclohexanone mise en œuvre à l'acide percarboxylique compris entre 0,50 et 0,90, l'acide carboxylique non transformé, l'excès d'acide percarboxylique et le solvant entraîneur azéotropique sont séparés par distillation du mélange réactionnel après l'oxydation et recyclés à l'étape de synthèse de l'acide percarboxylique et l'ε-caprolactone brute résultant de la distillation précédente purifiée par une simple évaporation sous pression réduite.

2. Procédé selon l'une quelconque des revendications 1 caractérisé en ce que l'acide percarboxylique mis en œuvre est l'acide perpropionique.

3. Procédé selon l'une quelconque des revendications 1 à 2, caractérisé en ce que le solvant entraîneur azéotropique est le dichloro-1,2-éthane.

## Claims

1. Process for manufacture of ε-caprolactone by oxydation of cyclohexanone, by means of a crude solution of $C_2$-$C_4$-percarboxylic acid resulting from the reaction of hydrogen peroxide on the corresponding carboxylic acid in the presence of a boric acid as catalyst with continuous removal of water by azeotropic entrainment, characterised in that the reaction between the cyclohexanone and the percarboxylic acid is carried out with a molar ratio of cyclohexanone used to percarboxylic acid of between 0.50 and 0.90, the unconverted carboxylic acid, the excess percarboxylic acid and the azeotropic entraining solvent are separated by distillation of the reaction mixture after the oxidation and recycled to the stage in which the percarboxylic acid is synthesised, and the crude ε-caprolactone resulting from the above distillation is purified by simple evaporation under reduced pressure.

2. Process according to claim 1, characterised in that the percarboxylic acid used is perpropionic acid.

3. Process according to either one of claims 1 to 2, characterised in that the azeotropic entraining solvent is 1,2-dichloroethane.

## Patentansprüche

1. Verfahren zur Herstellung von ε-Caprolacton durch Oxydation von Cyclohexanon mittels einer rohen Lösung einer $C_2$-$C_4$-Percarbonsäure, die bei der Reaktion von Wasserstoffperoxid mit der entsprechenden Carbonsäure in Gegenwart einer Borsäure als Katalysator unter kontinuierlicher Entfernung des Wassers durch azeotropes Mitschleppen entsteht, dadurch gekennzeichnet, daß die Reaktion zwischen dem Cyclohexanon und der Percarbonsäure mit einem molaren Verhältnis des eingesetzten Cyclohexanons zur Percarbonsäure zwischen 0,50 und 0,90 durchgeführt wird, daß die nicht umgewandelte Carbonsäure, der Überschuss an Percarbonsäure und das azeotrope Schleppmittel nach der Oxydation aus dem Reaktionsgemisch durch Destillation abgetrennt und zur Synthesestufe der Percarbonsäure zurückgeführt werden und daß das rohe ε-Caprolacton, das bei der vorgenannten Destillation erhalten wird, durch eine einfache Verdampfung unter vermindertem Druck gereinigt wird.

2. Verfahren nach Anspruch 1, dadurch gekennzeichnet, daß die eingesetzte Percarbonsäure Perpropionsäure ist.

3. Verfahren nach einem der Ansprüche 1 oder 2, dadurch gekennzeichnet, daß das azeotrope Schleppmittel das 1,2-Dichlorethan ist.

0 084 286